# EUROPEAN PATENT APPLICATION

(11) **EP 1 108 364 A2**
(43) Date of publication of application: **20.06.2001**
(21) Application number: 00311172.1
(22) Date of filing: 14.12.2000
(51) Int. Cl.: A23D 9/007, A23L 1/24, A23L 1/30

(54) **Long chain alcohols admixed in sterol compounds**

(30) Priority: 15.12.1999 US 461607
(71) Applicant: McNEIL-PPC, INC., Skillman, NJ 08858 (US)
(72) Inventor: Dartey, Clemence K., North Wales, PA 19545 (US); Sox, Thomas E., Ambler, PA 19002 (US)
(74) Representative: Mercer, Christopher Paul

(57) **Abstract**

The present invention provides a method of providing a desirable admixture of long chain alcohols and sterols in a food or beverage product. More specifically, this process comprises dissolving or suspending the long chain alcohol in the sterol, and then incorporating the admixture into food or beverage product.

## Description

### Related Applications

This application is being concurrently filed with two additional applications. These related applications are entitled Long Chain Alcohols Provided in Edible Oils, European Patent Application No. , claiming priority from USSN 09/461 887 (Attorney's ref: P026182EP) European Patent Application No. , claiming priority from USSN 09/461 592 (Attorney's ref: P026184EP).

### Field of the Invention

The present invention relates to the incorporation of long chain alcohol substances, more specifically the present invention relates to the encapsulation of long chain alcohols so as to more readily incorporate them into foods.

### Background of the Invention

Long chain alcohols, including policosanol, are therapeutically useful materials with efficacy in lowering cholesterol or blood lipids, inhibiting platelet aggregation and improving stamina. Therefore, the incorporation of long chain alcohols such as policosanol in various foods is desirable.

Incorporation of policosanol into high fat or fat-continuous emulsion systems such as margarine and margarine spreads is complicated by the functional properties of policosanol. In particular, incorporation of policosanol into a margarine oil system containing diglycerides and a phospholipid causes an increase in the hardness of a margarine type product as disclosed in WO 98/47385. In addition, EP 0991804 discloses that the incorporation of a natural long chain alcohol in a fat continuous system reduces the viscosity and yield values of confectionery products.

Despite these disclosures there is still an ongoing need to be able to add long chain alcohols to foods in a manner that the long chain alcohol will be easily formulated, will remain stable during storage, and will not adversely affect the properties of the food.

### Summary of the Invention

The present invention provides comestibles that contain an admixture of long chain alcohols and sterol compounds. Both the long chain alcohols and sterol compounds are provided in the amounts effective to lower cholesterol levels in a human being when consumed on a regular basis.

### Detailed Description of the Invention

The present invention is directed to the admixture of long chain alcohols into sterol compounds and the incorporation of the alcohol/sterol mixtures into food or pharmaceutical products. By long chain alcohols, it is understood to mean both saturated and unsaturated alcohols of C₂₀ and above, primarily from C₂₀ to C₃₆. As used herein, long chain alcohols are understood to be those materials that contain more than about 90 weight percent C₂₀ or longer, primarily aliphatic alcohol materials. For the greatest health benefit it is preferred that the long chain alcohols be more than 50 weight percent octacosanol (C₂₈), preferably more than 65 percent, more preferably greater than about 70 weight percent. As used herein policosanol is understood to be a mixture of long chain alcohols ranging from C₂₀ to C₃₆ with greater than 50 weight percent C₂₈, preferably greater than 65 weight percent octacosanol. Common distribution and concentration ranges of the various components of policosanol are disclosed in US Patent 5,856,316, hereby incorporated by reference. These long chain alcohols are available from various natural sources, most preferably from sugar cane wax. The long chain alcohols can also be synthesized using techniques well known in the art.

The level of long chain alcohols incorporated in a food is determined by serving size of the food product. It is desirable to provide in a single serving an effective amount of the long chain alcohols to derive the desired physiological benefits, such as reduced platelet aggregation, reduced lipid and cholesterol levels and the like. Typically the level of the long chain alcohols is from about 0.5 to about 400 mg/serving size; preferably from about 1.0 to about 100 mg/serving and most preferably from about 2.0 to about 20 mg/ serving size of a food product. When used in pharmaceutical dosage forms, it is preferred that the level of the long chain alcohol is less than about 40 milligrams, preferably from about 2 to about 20 milligrams so as to maintain a stable dosage form.

There are many foods into which the encapsulated long chain alcohols are incorporated, including but not limited to margarine, spreads, salad dressings, beverages and juices, cookies, confectionery products, creams, cheeses, oils, candy and the like.

The sterol materials in which the long chain alcohols are dissolved are well known in the art. As used herein the term sterol is understood to include cholesterol-lowering materials including sterols, stanols, stanol esters, sterol esters. Among the preferred materials are β-sitosterol and β-sitostanol, as well as oryzanol and mixtures of various sterols and stanols. These materials are well known in the art and are disclosed in U.S. Patent Nos. 3,881,005; 5,502,045; 5,869,708, hereby incorporated by reference and WO 98/19556. The level of sterols used in the present invention should be sufficient to provide an amount effective in lowering cholesterol in a vertebrate, such as mammals, reptiles and amphibians, preferably a human when consumed on a routine basis. Typically the sterol level is from about 0.4 to about 4.0 g, preferably from about 0.8 to about 3.0 g, and most preferably from about 1.0 to about 2.0 g per serving.

The relative weight ratios of the long chain alcohols and the sterol compounds are such that the long chain alcohols will be admixed into the sterol compounds. In a preferred embodiment, the admixture is then heated to melt completely before adding the liquid blend to the oil phase or the aqueous phase compositions. In preparing a cholesterol-lowering margarine or a margarine spread, the long chain alcohols are mixed with melted stanol esters at about 140 °F. The mixture is heated to 180 °F to dissolve completely the long chain alcohols. The hot alcohol/stanol ester preparation is added to the margarine oil stabilized at 160 °F before the aqueous phase is mixed with the oil phase to produce the margarine emulsion. In a preferred embodiment of this invention, the mixture of sterol compounds and long chain alcohols are heated and dissolved in a portion of the margarine oil at about 180 °F, This oil blend is then added to remaining oil phase maintained at 160 °F prior to the addition of the aqueous phase to form the margarine emulsion. At temperatures above 140 °F, policosanol preparations, as high as 1.0 %, remain dissolved in stanol esters, sterol esters, sterols, stanols and the like.

Emulsifying agents and surfactants can also be employed to aid in admixing the long chain alcohols in the sterol compounds. Suitable emulsifying agents and suspending agents include any food grade materials, such as mono and diglycerides, TWEENS and SPANS, lecithin, polyglycerol esters, propylene glycol esters and the like, polysorbates, mono and diglycerides of fatty acids, sucrose fatty acid esters and polyoxyethylene derivatives of sorbitan fatty acid esters. These surfactants are well known in the art and are commercially available.

Suitable polyglycerol esters include triglyceryl monostearate, hexaglyceryl distearate, hexaglyceryl monopalmitate, hexaglyceryl dipalmitate, decaglyceryl distearate, decaglyceryl monooleate, decaglyceryl dioleate, decaglycerol monopalmitate, decaglycerol dipalmitate, decaglyceryl monostearate, octaglycerol monooleate, octaglycerol monostearate and decaglycerol monocaprylate.

Other useful surfactants include polysorbates made from the reaction product of monoglycerides or sorbitan esters with ethylene oxides. Examples of useful polysorbates include polyoxyethylene 20 mono- and diglycerides of saturated fatty acids, polyoxyethylene 4 sorbitan monostearate, polyoxyethylene 20 sorbitan tristearate, polyoxyethylene 20 sorbitan monooleate, polyoxyethylene 5 sorbitan monooleate, polyoxyethylene 20, sorbitan trioleate, sorbitan monopalmitate, sorbitan monolaurate, propylene glycol monolaurate, glycerol monostearate, diglycerol monostearate, glycerol lactyl-palmitate.

Other suitable surfactants with HLB values provided in brackets, [ ], include decaglycerol monolaurate[15.5]; decaglycerol distearate [10.5]; decaglycerol dioleate [10.5]; decaglycerol dipalmitate [11.0]; decaglycerol monostearate [13.0]; decaglycerol monooleate [13.5]; hexaglycerol monostearate [12.0]; hexaglycerol monooleate [10.5]; hexaglycerol monoshortening [12.0]; polyoxyethylene (20) sorbitan monolaurate [16.7]; polyoxyethylene (4) sorbitan monolaurate [13.3]; polyoxyethylene (20) sorbitan monopalmitate [15.6]; polyoxyethylene (20) sorbitan monostearate [14.9]; polyoxyethylene (20) sorbitan tristearate [10.5]; polyoxyethylene (20) sorbitan monooleate [15.0]; polyoxyethylene (5) sorbitan monooleate [10.0]; polyoxyethylene (20) sorbitan trioleate [11.0]. As is appreciated by those with skill in the art, the HLB value for a surfactant is an expression of its Hydrophile-Lipophile Balance, i.e., the balance of the size and strength of the hydrophilic (polar) and lipophilic (non-polar) groups of the surfactant.

Lactic acid derivatives include sodium stearoyl lactylate and calcium stearoyl lactylate. Most preferably the emulsifying materials are polysorbates and the partial glycerides, including mono and diglycerides.

Another technique which can be employed is to melt and dissolve both the long chain alcohol and sterol compound in a suitable solvent. Any suitable food-grade vegetable fats and oils can be employed as solvents for the long chain alcohols and the sterol compounds. Aqueous based systems can also be employed, but this will require the use of various emulsifying agents approved for food applications as set forth above.

In a highly preferred embodiment, other ingredients which have desirable physiological actions can be added to the food products. Such materials include soy, vitamins, minerals and the like. Also included in the comestible are other ingredients typically used in the preparation of foods which include without limitation, flavors, colors, preservatives, sweeteners and the like.

US Patent No. 5,952,393 (Sorkin) discloses tablet and soft gelatin compositions containing an impure policosanol obtained from rice bran and sterol blend. In contrast, the present invention is directed to the incorporation of an intimate admixture of long chain alcohol and phytosterols into a food or beverage product. Sterols are believed to lower serum cholesterol levels by blocking the absorption of dietary cholesterol and preventing the resorption of bile acids secreted during the digestive process. Therefore sterols are most efficacious when incorporated in the form of a food or beverage product. When incorporated into a food or beverage, the sterols are intimately mixed with the other ingredients of the food into which they are formulated. Additionally the process of mastication and gastric mixing provides mixing of the sterols with cholesterol containing food that may be ingested in the same meal.

Sorkin also discloses the use of from about 58 to 83 milligrams of rice bran wax derived policosanol per tablet or soft gelatin capsule. In contrast, the present invention is directed to the administration of about 2 to about 20 milligrams of long chain alcohol, preferably policosanol per serving. The lower levels of the long chain alcohols are still therapeutically effective, particularly when consumed in combination with sterol compounds. Another advantage of the present invention is that the long chain alcohol/sterol combination is stable. Stable, as used herein means that the long chain alcohol does not precipitate or separate from the sterol compound.

The following examples are provided as specific embodiments of the present invention. Other modifications of this invention will be readily apparent to those skilled in the arts without departing from the scope of the present invention. The source of the material used in an example is provided in parenthesis.

### Example 1

This example discloses the application of policosanol with stanol esters in a margarine spread formulation. The mixture of policosanol and the sterol derivative is stirred and heated in a hot water bath to 180 °F. The clear policosanol preparation is added to the oil phase components of the cholesterol-lowering margarine spread.

**Table 1:**

| Cholesterol Lowering Margarine Spreads Containing Policosanol Dissolved in Sterol Derivatives | | | | |
|---|---|---|---|---|
| Ingredients | Stanol Esters Weight (lbs.) | terol Esters eight (lbs.) | tanols eight lbs.) | terols eight lbs.) |
| Liquid Soybean Oil | 11.7923 | 1.7923 | 1.7923 | 1.7923 |
| Partially Hydrogenated Soybean Oil | 11.0000 | 1.0000 | 5.0000 | 5.0000 |
| Canola Oil | 30.0000 | 0.0000 | 5.0510 | 5.0510 |
| Plant Stanol Esters | 21.5500 | | | |
| Plant Sterol Esters | - | 1.5500 | | |
| Plant Stanols | - | | 2.4990 | |
| Plant Sterols | - | | | 2.4990 |
| Monoglycerides | 0.3000 | .3000 | .3000 | .3000 |
| Lecithin | 0.2000 | .2000 | .2000 | .2000 |
| Hexaglycerol Distearate, Polyaldo 6-2-S | 0.1000 | .1000 | .1000 | .1000 |
| Artificial Butter Flavor | 0.0500 | .0500 | .0500 | .0500 |
| Vitamins A and D | 0.00625 | .00625 | .00625 | .00625 |
| Beta Carotene 30 % Solution | 0.00150 | .00150 | .00150 | .00150 |
| Policosanol, 93.16 % Active, Garuda International, CHOLESSTANOL | 0.0679 | .0679 | .0679 | .0679 |
| Aqueous Phase | | | | |
| Water | 22.8175 | 2.8175 | 2.8175 | 2.8175 |
| Salt | 2.0000 | .0000 | .0000 | .0000 |
| Citric Acid | 0.0075 | .0075 | .0075 | .0075 |
| Calcium Disodium EDTA | 0.0070 | .0070 | .0070 | .0070 |
| Potassium Sorbate | 0.1000 | .1000 | .1000 | .1000 |
| Total | 100.000 | 00.000 | 00.000 | 00.000 |

The margarine emulsion was prepared in a pilot plant by blending the aqueous phase with the oil phase. The oil phase was prepared by blending together the liquid soybean oil, partially hydrogenated soybean oil, canola oil, monoglycerides, lecithin and hexaglycerol distearate in a jacketed margarine emulsion tank. The tank was heated with hot water to 160 °F to melt and produce a clear liquid oil blend.

Policosanol was mixed with stanol esters in a stainless steel container. The container was placed in a water-bath on a stove and heated to approximately 180 °F to melt. The clear liquid policosanol preparation was transferred and mixed with the oil blend in the emulsion tank. The butter flavor, vitamins A and D blend and beta-carotene color were blended with the oil to complete the oil phase component of the margarine spread. The aqueous phase was prepared by dissolving salt, citric acid, ethylenediaminetetra acetic acid (EDTA) and potassium sorbate in the water. To prepare the margarine emulsion, the emulsion tank agitator was turned on at high speed and the aqueous phase was blended slowly into the oil phase to form the margarine emulsion. The temperature of the margarine emulsion was adjusted to 130 ± 5 °F. The margarine spread emulsion was pumped and chilled in a scrape surface heat exchanger, through to a pin worker to a filling unit. The temperature of the margarine spread at the filling unit was maintained at 45 - 65 °F by adjusting the temperature of the emulsion in the scrape surface heat exchanger. The finished margarine was packed in suitable containers and stored refrigerated. Each margarine formulation contained approximately 5 mg policosanol per 8 gram serving size.

### Example 2

This example discloses cholesterol-lowering light margarine spreads containing policosanol dissolved in sterol derivatives.

**Table 2:**

| Cholesterol-Lowering Light Margarine Spreads Containing Policosanol Dissolved in Sterol Derivatives | | | | |
|---|---|---|---|---|
| Ingredients | tanol Esters | Sterol Esters % | Stanols % | Sterols % |
| Liquid Soybean Oil | 7.9913 | 7.9913 | 37.0423 | 37.0423 |
| Plant Stanol Esters | 1.5500 | | - | - |
| Plant Sterol Esters | | 1.5500 | - | - |
| Plant Stanols | | | 12.4990 | - |
| Plant Sterols | | | - | 12.4990 |
| Mono-glycerides | .4000 | .4000 | .4000 | .4000 |
| Lecithin | .3000 | .3000 | .3000 | .3000 |
| Hexaglycerol Distearate, Polyaldo 6-2-S | .1000 | .1000 | .1000 | .1000 |
| Hexaglycerol Mixed Esters, CAPROL ET | .1000 | .1000 | .1000 | .1000 |
| Artificial Butter Flavor | .0500 | .0500 | .0500 | .0500 |
| Vitamins A and D | .00625 | .00625 | .00625 | .00625 |
| Beta Carotene 30 % Solution | .00250 | .00250 | .00250 | .00250 |
| Policosanol, 93.16 % Active, Garuda CHOLESSTANOL | .0679 | .0679 | .0679 | .0679 |
| Aqueous Phase | | | | |
| Water | 7.3150 | 7.3150 | 7.3150 | 7.3150 |
| Salt | .0000 | .0000 | .0000 | .0000 |
| Potassium Sorbate | .1000 | .1000 | .1000 | .1000 |
| Citric Acid | .0100 | .0100 | .0100 | .0100 |
| Calcium Disodium EDTA | .0070 | .0070 | .0070 | .0070 |
| Total | 00.000 | 00.000 | 00.000 | 00.000 |

Liquid soybean oil, partially hydrogenated vegetable oil, monoglycerides, lecithin, hexaglycerol distearate and hexaglycerol mixed esters were mixed together in a margarine emulsion tank and heated to 160 °F. Policosanol and stanol esters were mixed together in a stainless steel container and heated in a hot water bath to 180 °F. The melted policosanol preparation was blended into the oil in the emulsion tank. The butter flavor, Vitamins A & D blend and beta-carotene color were mixed into the oil to make up the oil phase. The water phase was prepared by dissolving the salt, potassium sorbate, citric acid and EDTA in the water. While mixing the oil phase at high speed, the aqueous phase was added slowly to form the margarine spread emulsion. The margarine emulsion was processed into a finished margarine spread by processing the emulsion through a scrape surface heat exchanger, through a pin worker or a B-unit through to a filler unit. The temperature of the chilling unit (the scrape surface heat exchanger) was regulated to produce a finished light margarine spread with a temperature of approximately 45 - 65 °F at the filler unit. The finished light margarine spreads were packed in suitable containers and stored refrigerated. Each light margarine spread formulation shown in Table 2 contained approximately 5 mg policosanol per 8 gram serving size.

### Example 3

This example concerns the use of stanol esters (and or sterol esters) as a carrier system for policosanol application in dressings. Policosanol was heated to dissolve in liquid stanol esters; or policosanol was mixed with solid stanol esters and heated to melt and form a clear liquid. The liquid policosanol preparation was then used to produce cholesterol lowering dressings e.g., ranch, French, creamy Italian, thousand island and mayonnaise dressings.

**Table 3:**

| Cholesterol-Lowering Ranch Dressings Containing (a) Policosanol and Stanol Esters and (b) Policosanol and Sterol Esters | | |
|---|---|---|
| | (a) | (b) |
| | Stanol Esters | Sterol Esters |
| Ingredients | Weight (g) | Weight (g) |
| Soybean Oil, Salad Oil | 480.00 | 480.00 |
| Vinegar, 120 Grain, White distilled | 84.00 | 84.00 |
| Stanol Esters | 71.34 | - |
| Sterol Esters | - | 71.34 |
| Sugar, Fine Granulated | 58.80 | 58.80 |
| Ranch Seasoning | 50.40 | 50.40 |
| Cultured Buttermilk Solids, Armour | 19.20 | 19.20 |
| Salt | 8.40 | 8.40 |
| CAPROL ET, A. C. Humko | 7.44 | 7.44 |
| Egg Yolk, Liquid, NEPA #2, 10 % Salt | 4.32 | 4.32 |
| KELTROL T Xanthan Gum, Kelco | 4.20 | 4.20 |
| Polysorbate 60 | 3.60 | 3.60 |
| Lemon Juice Concentrate, 400 GPL | 3.00 | 3.00 |
| Propylene Glycol Alginate, KELCOLOID LVF, Kelco | 2.10 | 2.10 |
| Polysorbate 80, TWEEN 80 | 1.92 | 1.92 |
| Titanium Dioxide | 1.68 | 1.68 |
| Potassium Sorbate | 0.96 | 0.96 |
| Sodium Benzoate | 0.96 | 0.96 |
| Vitamin E Acetate Roche | 0.22 | 0.22 |
| Policosanol, 95 % Active, Garuda Cholesstanol | 0.42 | 0.42 |
| DL-Alpha-Tocopherol | 0.10 | 0.10 |
| Calcium Disodium EDTA | 0.07 | 0.07 |
| Paprika Oleoresin 1000 ASTA | 0.02 | 0.02 |
| Water | 396.85 | 396.85 |
| TOTAL: | 1200.00 | 1200.00 |

The ranch dressings were prepared using a POWERGEN homogenizer as the mixing unit. The preservatives, sodium benzoate and potassium sorbate, and EDTA were dissolved in the water. A mixture of the hydrocolloid gums, xanthan gum and propylene glycol alginate, was dispersed in a portion of the vegetable oil (1 part gum in 2 - 5 parts of portion of the oil). The gum dispersion was blended and hydrated in the water at 8,000 rpm for 10 minutes. After blending in the vinegar and lemon juice, the sugar, buttermilk solids, ranch seasoning, salt and titanium dioxide were added and blended at 12,000 rpm for 5 minutes. The polysorbates, 60 and 80 were melted together and blended with the egg yolk in the aqueous phase for about 1 minute at 12,000 rpm.

Policosanol was added to melted stanol esters and heated in a water-bath to dissolve. The preparation was added to the vegetable oil/CAPROL ET blend at about 140 °F. While mixing the aqueous phase at high speed, 14,000-rpm minimum, the oil preparation was added slowly to the aqueous phase. During the addition of the oil phase, a mixture of the vitamin E acetate, alpha tocopherol and paprika oleoresin was added. After all the oil phase ingredients were added, the dressing emulsion was blended for four more minutes to produce the finished ranch dressing which was filled in bottles and stored. Each dressing contained approximately 10 mg policosanol per 30 gram serving size.

### Example 4

This example discloses cholesterol-lowering Thousand Island dressings containing policosanol with stanol esters and policosanol with sterol esters.

**Table 4:**

| Cholesterol-Lowering Thousand Island Dressings Containing (a) Policosanol with Stanol Esters and (b) Policosanol with Sterol Esters. | | |
|---|---|---|
| | (a) | (b) |
| Ingredients | atch Weight (g) | Batch Weight (g) |
| Soybean Oil, Salad Oil | 17.60 | 417.60 |
| Relish, Sweet, Ripon | 32.00 | 132.00 |
| Sugar, Fine Granulated | 26.00 | 126.00 |
| Vinegar, 120 Grain, White distilled | 0.40 | 80.40 |
| Tomato Paste 31 % Solids | 2.00 | 72.00 |
| Stanol Esters | 8.96 | - |
| Sterol Esters | | 68.96 |
| Salt | 8.00 | 18.00 |
| CAPROL ET, A. C. Humko | .36 | 6.36 |
| Mustard Flour, Baltimore Spice | .00 | 6.00 |
| Egg Yolk, Liquid, Nepa #2, 10 % Salt | .80 | 4.80 |
| Polysorbate 60 | .60 | 3.60 |
| KELTROL T Xanthan Gum, Kelco | .12 | 3.12 |
| Lemon Juice Concentrate, 400 GPL | .40 | 2.40 |
| Propylene Glycol Alginate, KELCOLOID LVF, Kelco | .56 | 1.56 |
| Polysorbate 80, TWEEN 80, | .20 | 1.20 |
| Onion, Minced | .84 | 0.84 |
| Sodium Benzoate | .60 | 0.60 |
| Potassium Sorbate | .20 | 1.20 |
| Garlic Powder | .40 | 2.40 |
| Black Aquaresin Pepper | .24 | 0.24 |
| Paprika | .24 | 0.24 |
| Vitamin E Acetate, Roche | .22 | 0.22 |
| Policosanol, 95 % Active, Garuda CHOLESSTANOL | .42 | 0.42 |
| Color, Caramel AP 100 Sethness | .12 | 0.12 |
| DL-Alpha-Tocopherol | .10 | 0.10 |
| Calcium Disodium EDTA | .07 | 0.07 |
| Water | 49.55 | 249.55 |
| TOTAL: | 200.00 | 1200.00 |

The preservatives (sodium benzoate, potassium sorbate and EDTA) were dissolved in the water. The gums (xanthan and the alginate) were dispersed in a portion of the salad oil (1 part gum in 2 - 5 parts salad oil). Using the POWERGEN homogenizer set at 8,000 rpm, the gum dispersion was blended in the water for 10 minutes. Vinegar and lemon juice were added and blended for 1 minute. Next, the sugar, tomato paste, salt, mustard flour, minced onion and garlic powder were added and blended at 12,000 rpm until the tomato paste was distributed uniformly in the preparation. The polysorbates, 60 and 80 were melted together and blended in the preparation. Liquid egg yolk and caramel color were then added and blended in for about 30 seconds.

Policosanol was added to the stanol esters. The mixture was heated and melted in a water-bath. The melted policosanol preparation was added to a blend of the salad oil, CAPROL ET, alphatocopherol, vitamin E acetate, black aquaresin pepper and paprika oleoresin maintained at about 140 °F. While blending the aqueous phase preparation at about 14,000 rpm, the oil blend was added slowly to form the dressing emulsion. After the oil addition was completed, the mixing was continued for two more minutes to obtain a tight emulsion. Sweet relish was then mixed gently in the emulsion to complete the process. The dressing was then filled into suitable containers for storage. Each dressing formulation contained approximately 10 mg policosanol per a serving size of 30 grams.

### Example 5

This example describes the application of policosanol dissolved in stanol esters and sterol esters in yogurts. Policosanol was mixed with melted stanol esters or sterol esters. The mixture was heated to approximately 160 - 180 °F in a water bath to melt and dissolve policosanol in the liquid stanol esters or sterol esters. The policosanol preparation was used to produce flavored yogurt products.

**Table 5:**

| Cholesterol-Lowering Yogurt Formulations Containing (a) Policosanol with Stanol Esters and (b) Policosanol with Sterol Esters. | | |
|---|---|---|
| | (a) | (b) |
| Yogurt Base Formulation | atch Weight (g) | Batch Weight (g) |
| Skim Milk | 3560.28 | 3560.28 |
| Liquid Cane Sugar | 451.22 | 451.212 |
| Condensed Skim Milk | 442.50 | 442.50 |
| Whole Milk (3.4% Milk Fat) | 369.00 | 369.00 |
| Stabilizer Blend (Germantown International) | 52.50 | 52.50 |
| MELOSKIM WP-75 Whey Protein Concentrate (Kerry Foods) | 50.00 | 50.00 |
| Stanol Esters (Raisio) | 47.42 | - |
| Sterol Esters (Raisio) | - | 47.42 |
| Kosher Edible Gelatin Powder (250) (GMI Products) | 16.25 | 16.25 |
| OPTA-MIST, Modified Food Starch (Opta Foods) | 10.00 | 10.00 |
| Policosanol 95 % Active, Garuda CHOLESSTANOL | 0.10 | 0.10 |
| Yogurt Culture YC087 (Chris Hansen) | 0.55 | 0.55 |
| Vitamin E, dl-Alpha-Tocopheryl Acetate (Roche) | 0.18 | 0.18 |
| TOTAL | 5000.00 | 5000.00 |

| Finished Fruit Flavored Yogurt | **(a)** | **(b)** |
|---|---|---|
| Ingredients | Batch Weight (g) | Batch Weight (g) |
| Yogurt Base Formulation | 4482.00 | 4482.00 |
| Fruit Preparation | 918.00 | 918.00 |
| TOTAL | 5400.00 | 5400.00 |
| Yogurt serving size, 8 oz (226.8 g) contained ~ 3.6 mg policosanol and ~ 1.0 g stanols or sterols. | | |

The laboratory process for preparing the yogurts was as follows. The liquid ingredients including the skim milk, whole milk, condensed milk and liquid sugar were blended together in a stainless steel container in a water-bath on a stove. The starch was dispersed in the liquid blend at medium speed (about 600 rpm). The stove was set at medium heat and the blend was heated slowly. The remaining dry ingredients including gelatin, stabilizers and whey protein concentrate were dispersed into the preparation and the blend was heated to 140 °F. Policosanol was added to stanol ester and heated to a clear liquid, approximately 160 - 180 °F. Vitamin E was added to the policosanol/sterol ester preparation and the mixture was blended with the milk preparation. The blend was heated in the water-bath to 190 °F. After holding the temperature at 190 °F for 5 minutes, the preparation was cooled and homogenized in two stages (1500/500 pounds per square inch) at approximately 140 °F. The homogenized preparation was cooled to about 109 °F and inoculated with the yogurt starter culture. The pH of the yogurt base was monitored hourly until pH 4.5 was attained. The preparation was agitated and cooled to 65 °F and stored overnight in a refrigerator. The finished yogurt was packed in suitable containers after mixing 83 parts of the yogurt base with 17 parts fruit flavor preparations.

### Example 6

This example discloses the application of policosanol dissolved in stanol esters or sterol esters in cholesterol-lowering pasteurized cheese spread formulation.

**Table 6:**

| Cholesterol-Lowering Pasteurized Cheese Spread Formulations Containing (a) Policosanol and Stanol Esters and (b) Policosanol and Sterol Esters. | | |
|---|---|---|
| | (a) | (b) |
| Ingredients | Batch Size (g) | Batch Size (g) |
| Light Cheddar Cheese (Cabot) | 1275.00 | 1275.00 |
| Stanol Esters (Raisio) | 287.50 | - |
| Sterol Esters (Raisio) | - | 287.50 |
| Policosanol, 95 % Active, Garuda CHOLESSTANOL | 0.62 | 0.62 |
| JOHA S-4, Sodium Phosphates (B K Ladenburg) | 50.00 | 50.00 |
| JOHA S-9 (Sodium Phosphates (B K Ladenburg) | 12.50 | 12.50 |
| Sodium Citrate | 6.25 | 6.25 |
| Nonfat Dry Milk Solids | 62.50 | 62.50 |
| Lactic Acid | 10.00 | 10.00 |
| Salt | 20.82 | 20.82 |
| Annatto Color (Chris Hansen) | 0.42 | 0.42 |
| Water | 774.39 | 774.39 |
| Total | 2500.00 | 2500.00 |

The laboratory process for preparing the cheese spread was as follows. The cheddar cheese was weighed in a stainless steel container. The container was heated in a water-bath on a stove to melt the cheese. Policosanol was added to stanol esters and heated in a water-bath to a clear liquid. The liquid policosanol preparation was transferred and blended with the melted cheddar cheese. About half the volume of the water was added and blended with the cheese preparation to a uniform consistency. The salt, nonfat dry milk solids, phosphates, citrate, lactic acid, annatto color and remaining water were added and blended to uniform consistency. The temperature of the cheese preparation was raised and held at 176 °F for 5 minutes. The finished product was packed hot into suitable containers and stored refrigerated. Each formulation contained approximately 3.5 mg of policosanol per 15 g of the processed cheese spread.

### Example 7

This example concerns the application of policosanol dissolved in stanol esters or sterol esters in preparing a cholesterol-lowering processed cheese sauce.

**Table 7:**

| Cholesterol-Lowering Processed Cheese Sauce Containing (a) Policosanol and Stanol Esters and (b) Policosanol and Sterol Esters. | | |
|---|---|---|
| | (a) | (b) |
| Ingredients | Batch Weight (g) | Batch Weight (g) |
| Light Cheddar Cheese (Cabot) | 1075.00 | 1075.00 |
| Stanol Esters (Raisio) | 287.50 | - |
| Sterol Esters (Raisio) | - | 287.50 |
| Policosanol, 95 % Active, Garuda CHOLESSTANOL | 0.62 | 0.62 |
| Sodium Citrate | 62.50 | 62.50 |
| Nonfat Dry Milk Solids | 83.25 | 83.25 |
| Lactic Acid | 10.00 | 10.00 |
| Salt | 25.00 | 25.00 |
| Annatto Color (Chris Hansen) | 0.42 | 0.42 |
| Water | 955.71 | 955.71 |
| Total | 2500.00 | 2500.00 |

The cheddar cheese sauce was prepared as follows. The cheddar cheese was melted in a suitable container placed in a water-bath heated on a stove. Policosanol was added to the stanol or sterol esters. The container was heated to about 160 - 180 °F in a water-bath to melt and dissolve policosanol in the stanol or sterol esters. The liquid policosanol preparation was blended with the melted cheese. About half the volume of the water was added to the melted cheese and blended to uniform consistency. After adding and mixing in the salt, nonfat milk solids, citrate, lactic acid and annatto color, the remaining water was added and blended to uniform consistency. The cheese sauce was then heated and the temperature held for five minutes at 176 °F. The finished cheese sauce was packed hot in suitable containers and stored refrigerated. Each formulation contained approximately 3.5 mg of policosanol per 15 g of the processed cheese sauce.

### Example 8

This example discloses the application of policosanol dissolved in stanol or stanol esters in typical cholesterol-lowering nutritional bars.

**Table 8:**

| Cholesterol-Lowering Chocolate Coated Nutritional Bar Formulations Containing (a) Policosanol and Stanol Esters and (b) Policosanol and Sterol Esters. | | |
|---|---|---|
| | (a) | (b) |
| Ingredients | Batch Weight (g) | Batch Weight (g) |
| Corn Syrup, 63 Dextrose Equivalents (Cargill) | 390.36 | 390.36 |
| KRYSTAR Crystalline Sugar (Far Best Foods) | 70.20 | 70.20 |
| CENTROPHASE C Lecithin (Central Soya) | 4.80 | 4.80 |
| Stanol Esters | 61.44 | - |
| Sterol Esters | - | 61.44 |
| Policosanol, 95 % Active, Garuda CHOLESSTANOL | 0.13 | 0.13 |
| Crisp Rice | 99.18 | 99.18 |
| Cocoa Powder (Paragon) | 76.00 | 76.00 |
| Oats, Baby | 73.40 | 73.40 |
| Soy Flour, Nutrisoy | 73.40 | 73.40 |
| Centex Textured Soy Flour, Unfortified (Central Soya) | 64.00 | 64.00 |
| Rice Flour, Pregelatinized | 26.46 | 26.46 |
| Vitamin E, 25 % RDA Blend | 12.14 | 12.14 |
| Ricex Rice Bran | 9.60 | 9.60 |
| Chocolate Fudge Flavor | 9.12 | 9.12 |
| Chocolate Flavor | 4.74 | 4.74 |
| Caramel Flavor | 1.09 | 1.09 |
| Target Coating | 223.94 | 223.94 |
| Total | 1200.00 | 1200.00 |

The corn syrup, sugar and lecithin were blended together in a stainless steel container and heated gently in a water-bath on a stove. The stanol esters and policosanol were mixed together and heated to melt at about 160 - 180 °F. The clear liquid policosanol preparation was blended gently into the hot syrup at about 160 °F. Vitamin E, the chocolate fudge, chocolate and caramel flavors were blended in the syrup preparation. The syrup preparation was transferred into a bench top Hobart bowl with a dough spindle mixer. The remaining dry ingredients were mixed together in a plastic container. The mixture was mixed gently at speed 1 in the syrup preparation to a uniform consistency. The dough was transferred into a pan and rolled with a wooden roller to desired thickness. The dough was covered with a wax paper and cooled in a refrigerator to firm up before cutting it into pieces. The pieces were then enrobed with the target coating and chilled through a cooling tunnel. The finished nutritional bars were then wrapped in foil and heat-sealed. Each formulation contained approximately, 3.5 mg of policosanol per 34 g serving size of the nutritional bar.

## Claims

1. A comestible comprising:
an effective amount of a long chain alcohol or mixture of long chain alcohols sufficient to lower cholesterol in a vertebrate, said long chain alcohol or alcohols being admixed with an effective amount of a sterol compound sufficient to lower cholesterol in a vertebrate, said long chain alcohol/sterol composition admixture being provided in a foodstuff.

2. The comestible of claim 1 wherein the amount of long chain alcohol is from 0.1 to 40 milligrams per serving, preferably from 1.0 to 20 milligrams per serving.

3. The comestible of claim 1 or claim 2 wherein the long chain alcohol is predominately octacosanol providing about 10 milligrams of octacosanol per serving.

4. The comestible of any one of claims 1 to 3 wherein the sterol compound is provided in an amount from 0.4 to 4.0 grams per serving.

5. The comestible of any one of claims 1 to 4 wherein the sterol compound is a sterol, stanol, sterol ester, stanol ester or oryzanol.

6. The comestible of claim 1 which is an oil, such as a cooking oil, a margarine or a salad dressing.

7. A method of preparing a comestible comprising:
providing a long chain alcohol in an amount sufficient to lower cholesterol in human;
providing a sterol compound in an amount sufficient to lower cholesterol in a human;
admixing said long chain alcohol and said sterol compound; and adding said admixture to a comestible.

8. The method of claim 7 wherein the long chain alcohol and sterol are heated.

9. The method of claim 7 or claim 8 wherein the admixture additionally contains a surfactant and/or an emulsifying agent.

10. The method of any one of claims 7 to 9 wherein the long chain alcohol and sterol admixture forms an emulsion.

11. The comestible of any one of claims 1 to 6 which contains less than about 40 mg of a long chain alcohol and 0.4 to 4 grams of a sterol for use in treating hypercholesterolemia or for reducing cholesterol in a vertebrate.
